# EUROPÄISCHE PATENTANMELDUNG

(11) **EP 4 714 487 A1**
(43) Veröffentlichungstag der Anmeldung: **25.03.2026**
(21) Anmeldenummer: 24201447.0
(22) Anmeldetag: 19.09.2024
(51) Int. Cl.: A61M 39/28

(54) **INFUSIONSPUMPENSYSTEM MIT SPEKTRALKODIERUNG**

(71) Anmelder: B. Braun Melsungen AG, 34212 Melsungen (DE)
(72) Erfinder: Schwarz, Jan, 34212 Melsungen (DE); Herwig, Dieter, 34212 Melsungen (DE); Sielemann, Martin, 34466 Wolfhagen (DE); Vogel, Markus, 37191 Katlenburg-Lindau (DE)
(74) Vertreter: Tergau & Walkenhorst Intellectual Property GmbH

(57) **Zusammenfassung**

Hauptanspruch: Infusionspumpensystem (10), umfassend:
• einen Artikel (4) eines Infusionssets,
• eine Spektralkodierung auf dem Artikel (4) als Identifikationsmerkmal,
• mindestens ein zusätzliches Identifikationsmerkmal (14) auf dem Artikel (4) ausgewählt aus einer Gruppe bestehend aus geometrischen Merkmalen, Aufdrucken, Lochmustern oder spezifischen Konturformen,
• ein Sensorsystem (20), das sowohl die Spektralkodierung als auch das zusätzliche Identifikationsmerkmal (14) erkennen und auswerten kann.

## Beschreibung

Die vorliegende Erfindung betrifft ein Infusionspumpensystem, das zur sicheren und eindeutigen Identifikation von Artikeln, insbesondere Einmalartikeln, in einem Infusionsset entwickelt wurde.

Infusionspumpensysteme spielen eine wichtige Rolle in der medizinischen Versorgung, indem sie eine präzise und kontinuierliche Verabreichung von Flüssigkeiten, Medikamenten oder Nährstoffen ermöglichen. Auswechselbare Artikel eines sogenannten Infusionssets wie Schlauchklemmen, Infusionsschläuche, Tropfkammern, Luftfilter, Adapter, Nadeln und Katheter sind neben den Infusionspumpen wesentliche Komponenten solcher Systeme. Die Identifikation und Kompatibilität dieser Artikel ist von großer Bedeutung für die Sicherheit und Effektivität der Infusionstherapie.

Bekannt ist die Identifikation von Artikeln eines Infusionssets durch eine Farbkodierung. Beispielsweise ist die Patentveröffentlichung DE 10 2021 215 067 A1 bekannt, in der eine Farbkodierung zur Identifikation von Schiebeklemmen beschrieben wird. Allerdings gibt es Einschränkungen bei der reinen Farberkennung, insbesondere hinsichtlich der Genauigkeit und der Vermeidung von Fehlanwendungen.

Die vorliegende Erfindung hat unter anderem die Aufgabe, ein Infusionspumpensystem zu schaffen, das die sichere und eindeutige Identifikation von Artikeln eines Infusionssets ermöglicht, um deren korrekte Verwendung zu gewährleisten und Fehlanwendungen zu verhindern.

Die genannte Aufgabe wird erfindungsgemäß gelöst durch ein Infusionspumpensystem gemäß Anspruch 1.

Demnach ist ein Infusionspumpensystem vorgesehen, umfassend:
- einen Artikel eines Infusionssets,
- eine Spektralkodierung auf dem Artikel als Identifikationsmerkmal,
- mindestens ein zusätzliches Identifikationsmerkmal auf dem Artikel ausgewählt aus einer Gruppe bestehend aus geometrischen Merkmalen, Aufdrucken, Lochmustern oder spezifischen Konturformen,
- ein Sensorsystem, das sowohl die Spektralkodierung als auch das zusätzliche Identifikationsmerkmal erkennen und auswerten kann.

Der Begriff "Spektralkodierung" ist hier als Verallgemeinerung des Begriffs "Farbkodierung" zu verstehen und umfasst diesen als Spezialfall. Gemeint ist eine "Farbkodierung", die neben sichtbaren Farben auch oder alternativ ausschließlich "Farben" (Wellenlängen oder Wellenlängenbereiche) außerhalb des sichtbaren Spektrums umfasst. Der Begriff "Spektralcodierung" bezieht sich somit auf die Zuweisung von Informationen zu bestimmten Bereichen des elektromagnetischen Spektrums, einschließlich sichtbarem Licht, Infrarot, Ultraviolett und anderen Wellenlängenbereichen. Als Spezialfall mit enthalten sind in diesem Begriff auch Fluoreszenzmarkierungen oder -kodierungen, die beispielsweise unter Beleuchtung mit UV-Licht (sogenanntes "Schwarzlicht") die energiereichen UV-Strahlen absorbieren und sie dann (durch Fluoreszenzanregung) als sichtbares Licht mit einer längeren Wellenlänge wieder abgeben. Eine derartige Spektralmarkierung ist mittels geeigneter optischer Sensoren, ggf. unter Zuhilfenahme von optischen Filtern (Farbfiltern etc.), maschinell erkennbar. Wie erwähnt ist als Spezialfall eine herkömmliche Farbkodierung im sichtbaren Bereich des elektromagnetischen Spektrums möglich, die (auch) ohne Hilfsmittel visuell erkennbar ist.

Die vorliegende Erfindung stellt ein verbessertes Infusionspumpensystem bereit, das zusätzlich zur Spektralkodierung mindestens ein weiteres Identifikationsmerkmal verwendet, um Artikel eines Infusionssets zu kennzeichnen und zu identifizieren. Dies erhöht die Sicherheit und Zuverlässigkeit der Identifikation und reduziert das Risiko der Verwendung inkompatibler oder nicht zugelassener Artikel.

Die zusätzlichen Identifikationsmerkmale sind bevorzugt in vier Hauptvarianten ausgeführt, die miteinander kombiniert werden können:
- Geometrische Merkmale: Stift oder Dorn oder zugehörige Aufnahme mit spezifischer Querschnittsform.
- Optische Merkmale: Aufdrucke, Prägungen oder Gravuren, insbesondere Lasergravuren, wie beispielsweise Strichcodes, QR-Codes oder andere zweidimensionale Codes.
- Lochmuster: Spezifische Muster, die durch Löcher, Durchgangsbohrungen oder Ausfräsungen im Artikel gebildet werden.
- Schlüsselbart-Konturen: Spezielle Konturformen, die ähnlich wie in einem Schloss mechanisch durch Abtastung detektiert werden.

Das zugehörige Detektorsystem ist zweckmäßigerweise auf das zusätzliche Erkennungsmerkmal abgestimmt und umfasst neben einem Spektralsensor (insbesondere Farbsensor) zur Erfassung der Spektralkodierung beispielsweise eine Kamera zur optischen Erfassung eines Aufdrucks, eine Lichtschrankenanordnung oder ein photosensitives Array zur Erkennung eines Lochmusters, oder eine mechanische Abtasteinheit zur Abtastung einer Schlüsselbar-Kontur. Das Detektorsystem kann beispielsweise in eine Infusionspumpe integriert sein oder als separate Komponente vorliegen.

Ein Vorteil der Erfindung ist die Vermeidung von Fehlanwendungen: Durch die Implementierung zusätzlicher Identifikationsmerkmale wird sichergestellt, dass nur die vorgesehenen und kompatiblen Artikel in das Infusionspumpensystem integriert oder verwendet werden. Dies reduziert das Risiko von Fehlern und gewährleistet, dass das System nur mit korrekten und sicheren Komponenten betrieben wird.

Durch das zusätzliche Identifikationsmerkmal ist eine redundante Identifikation ermöglicht. Das heißt, neben der Spektralkodierung werden zusätzliche Erkennungsmerkmale eingeführt, um eine redundante Erkennung zu ermöglichen. Dies erhöht die Genauigkeit der Identifikation und verringert die Möglichkeit, dass Fehler oder Störungen unbemerkt bleiben.

Des Weiteren ermöglicht das erfindungsgemäße System eine individuelle Anpassung: Die Erfindung ermöglicht es nämlich, Artikel eines Infusionssets spezifisch für verschiedene Anwendungen, Anwender oder Patienten zu kodieren. Dadurch kann das Infusionspumpensystem auf unterschiedliche medizinische Anforderungen und Nutzungsszenarien zugeschnitten werden.

Durch die Verwendung mehrerer Identifikationsmerkmale, wie geometrische Formen, Aufdrucke, Lochmuster oder spezifische Konturformen, können die Artikel vielseitig gestaltet werden, um verschiedene Einsatzmöglichkeiten abzudecken. Es ist daher ein hoher Grad an Individualisierung erreichbar.

Zusammengefasst zielt die Erfindung darauf ab, ein verbessertes Infusionspumpensystem bereitzustellen, das durch zusätzliche Identifikationsmerkmale eine zuverlässige und sichere Verwendung von Artikeln eines Infusionssets ermöglicht und die Anpassungsfähigkeit und Flexibilität des Systems erhöht.

Verschiedene Ausführungsbeispiele der Erfindung werden nachfolgend unter Bezugnahme auf die beigefügten Zeichnungen näher erläutert. Es zeigt:
- FIG. 1 - 4: jeweils eine Schlauchklemme (als Beispiel für einen Artikel eines Infusionssets) mit einer Farbkodierung und mit einem zusätzlichen Identifikationsmerkmal, und
- FIG. 5: rein schematisch ein Infusionspumpensystem mit einer Infusionspumpe, einem Sensorsystem und einem Artikel eines Infusionssets.

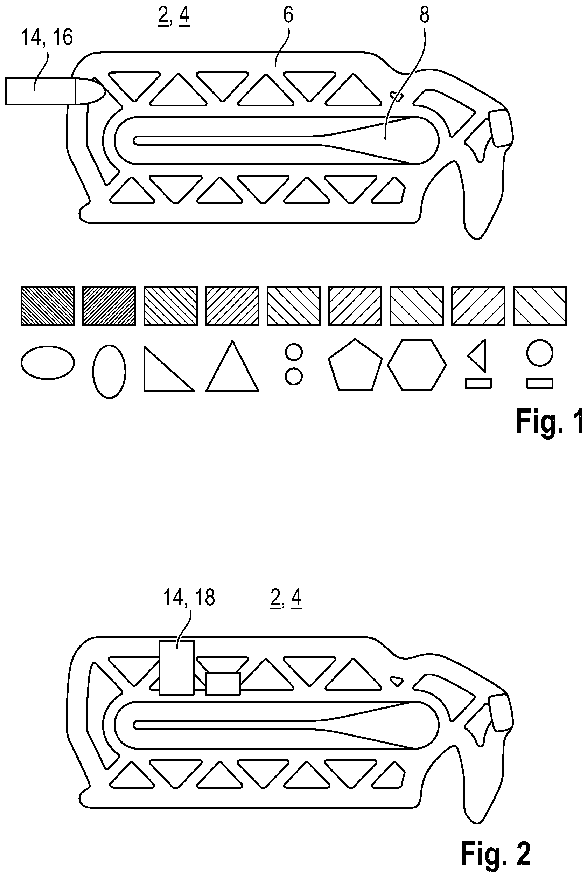

FIG. 1 bis FIG. 4 zeigen jeweils eine Schlauchklemme 2 als ein Beispiel für einen Artikel 4 oder Bestandteil eines Infusionssets. Typischerweise handelt es sich bei der Schlauchklemme 2 um einen Infusionseinmalartikel, der für den einmaligen Gebrauch konzipiert ist und nach der Anwendung entsorgt wird. Es kann sich aber auch um einen Mehrwegartikel handeln, der sterilisiert und mehrfach verwendet werden kann.

Schlauchklemmen 2 werden verwendet, um den Durchfluss in einem Infusionsschlauch zu regulieren oder zu stoppen. Hier im Beispiel weist die Schlauchklemme 2 einen Grundkörper 6 auf, der ein sich verjüngendes Langloch 8 umschließt. Beim Gebrauch der Schlauchklemme 2 wird ein Infusionsschlauch durch das Langloch 8 geführt. Befindet sich der Infusionsschlauch im breiten Ende des Langlochs 8, so wird er nicht zusammengedrückt und ist geöffnet. Wird der Infusionsschlauch zum anderen, schmalen Ende des Langlochs 8 geschoben, so wird er zusammengedrückt und verschlossen. Eine derart ausgebildete Schlauchklemme 2 wird auch als Schiebeklemme bezeichnet.

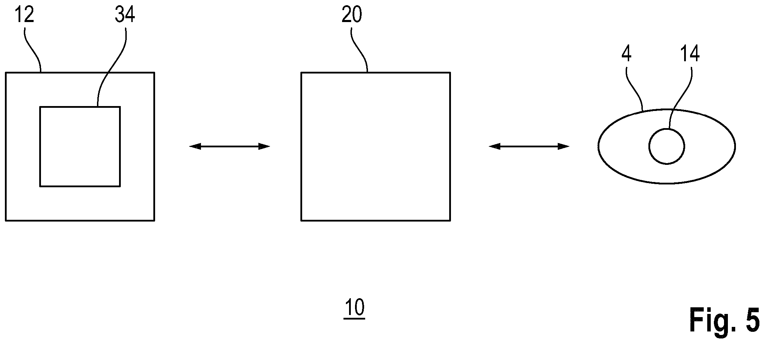

Um die Art oder den Typ der Schlauchklemme 2 identifizieren zu können, ist die Schlauchklemme hier im Beispiel mit einer Farbkodierung (als Spezialfall einer Spektralkodierung) versehen. In einer möglichen Realisierung ist der gesamte Grundkörper 6 oder nur ein Teilabschnitt davon an seiner Oberfläche spezifisch eingefärbt bzw. farbig ausgeführt. Die Zuordnung zwischen Farbe und Informationsgehalt kann in einer zugehörigen Farbtabelle hinterlegt sein. Die Farbkodierung ist vorteilhafterweise durch einen zugeordneten Sensor auslesbar, der Bestandteil eines Infusionspumpensystems 10 sein kann. Rein beispielhaft kann der Sensor in ein Klemmmodul einer Infusionspumpe 12 integriert sein, welches zur Aufnahme der Schlauchklemme vorgesehen ist (siehe auch FIG. 5 und die Beschreibung weiter unten).

Zur Vergrößerung des Informationsgehalts weist die Schlauchklemme 2 gemäß FIG. 1 bis FIG. 4 neben der Farbkodierung jeweils ein weiteres Erkennungsmerkmal (zusätzliches Identifikationsmerkmal 14) bzw. eine weitere Kodierung auf, das/die vorzugsweise sensorisch erfasst und überprüft werden kann. Dadurch lässt sich entweder die Gesamtmenge der kodierten Information erhöhen, oder eine in der einen Kodierung enthaltene Information kann für eine erhöhte Sicherheit redundant in der anderen Kodierung enthalten sein. Im letzteren Fall besonders bevorzugt ist diversitäre Redundanz, sprich eine mit anderen physikalischen Mitteln auslesbare Kodierung.

In der Variante gemäß FIG. 1 weist die Schlauchklemme 2 als weiteres Erkennungsmerkmal bzw. zusätzliches Identifikationsmerkmal 14 neben der Farbkodierung eine Aufnahme für einen Stift oder Dorn 16 mit spezifischer Querschnittsform auf (etwa kreisförmig, oval, dreieckförmig, viereckförmig, aus elementaren geometrischen Formen zusammengesetzt, komplex geformt, etc.). Nur wenn die Querschnitte von Stift oder Dorn 16 und zugehöriger Aufnahme zueinander passen, lässt sich der Stift oder Dorn 16 vollständig in die Aufnahme einstecken und dadurch Formschluss herstellen. Der Stift oder Dorn 16 kann rein beispielhaft Bestandteil einer Infusionspumpe 12 sein, in die die Schlauchklemme 2 zum Betrieb eingelegt wird.

Es kann aber auch umgekehrt ein Stift oder Dorn 16 mit spezifischer Querschnittsform in die Schlauchklemme 2 fest eingesetzt oder an sie angeformt oder anderweitig mit ihr verbunden sein und nach außen vom Grundkörper 6 abstehen, so dass der Stift oder Dorn 16 mit einer externen Aufnahme, beispielsweise an/in einer Infusionspumpe 12, wechselwirkt. Auch in diesem Fall ist durch die Formkodierung sichergestellt, dass nur "passende" Schlauchklemmen 2 verwendbar sind.

In einer Variante der vorigen Ausführungsform kann der Stift oder Dorn 16 auch selbst eine Farbkodierung Spektralkodierung tragen. Diese Farbkodierung, die neben der Formkodierung besteht, kann ergänzend oder auch alternativ zur Farbkodierung des Grundkörpers 6 vorgesehen sein. In FIG. 1 sind unterhalb der abgebildeten Schlauchklemme 2 unterschiedliche Farben und Querschnittsformen abgebildet, die sich beliebig kombinieren lassen.

In der Variante gemäß FIG. 2 ist neben der Farbkodierung ein dedizierter Teilabschnitt der Schlauchklemme 2 mit einem Aufdruck 18 versehen, der eine kodierte Information enthält. Besonders vorteilhaft aufbringen lässt sich ein "einfacher Siebdruck". Der einfache Siebdruck, auch als Serigrafie bekannt, ist ein Druckverfahren, bei dem Farbe durch ein feinmaschiges Gewebe (das Sieb) auf ein Substrat (hier: den Grundkörper 6 der Schlauchklemme 2) aufgetragen wird. Dieses Verfahren ermöglicht es, detaillierte und gleichmäßige Drucke zu erstellen, die sich für die Darstellung von Informationen eignen. Denkbar als aufgedruckter Informationsträger ist beispielweise ein Strichcode, ein QR-Code oder ein sonstiger zweidimensionaler Code.

Der Aufdruck 18 wird vorzugsweise durch eine zugeordnete Kamera (als Bestandteil eines Sensorsystems 20) erfasst und durch eine Auswerteinheit, die in die Kamera integriert oder ausgelagert sein kann, ausgewertet. Vorteilhafterweise ist der Kontrast von Aufdruck und Klemmenfarbe so hoch, dass mit einer schwarz-/weiß-Aufnahme und z. B. einem Kantenfilter gearbeitet werden kann, um den Aufdruck 18 zu erfassen und vom Rest der Schlauchklemme 2 zu unterscheiden.

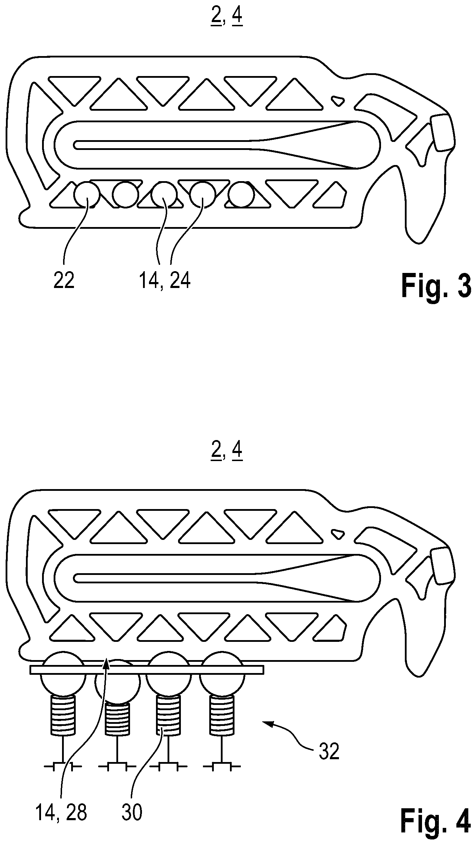

In der Variante gemäß FIG. 4 weist die Schlauchklemme 2 als weiteres Erkennungsmerkmal bzw. zusätzliches Identifikationsmerkmal 14 neben der Farbkodierung eine Anzahl von den Grundkörper 6 durchsetzenden Löchern 22 oder Durchgangsbohrungen oder Schlitzen auf, die zusammen ein spezifisches Lochmuster 24 bilden. Das Lochmuster 24 kann vorteilhafterweise optisch erfasst und hinsichtlich der darin kodierten Information ausgewertet werden kann. Die optische Abtastung kann auf vielfältige Weise erfolgen, z. B. mittels Lichtschranke, photosensitives Array, eindimensionaler Position Sensitive Detector (PSD), mehrdimensionaler Position Sensitive Detector, Charge Couple Device (CCD), Position Sensitive Phototransistor etc.

Wie in den anderen Fällen kann die entsprechende Detektoreinheit bzw. das Sensorsystem 20 beispielsweise in eine Infusionspumpe 12 oder ein anderes Gerät eines Infusionspumpensystems 10 integriert sein.

In der Variante von FIG. 4 schließlich ist durch geeignete Formgebung an einem Abschnitt der Außenkontur der Schlauchklemme 2 eine Art von Schlüsselbart-Kodierung oder Schlüsselbart-Kontur 28 verwirklicht. Ein Schlüsselbart ist normalerweise der untere, meist komplex geformte Abschnitt eines Schlüssels, der speziell dazu gestaltet ist, zugeordnete Stifte oder Hebel im Schloss in die richtige Position zu bringen. Analog dazu werden im vorliegenden Fall durch die genannte Formkodierung der Schlauchklemme 2 Stößel 30 einer zugeordneten Abtasteinheit 32 in eine entsprechende Position oder Stellung gebracht, welche sensorisch detektierbar ist, im einfachsten Fall etwa durch Schaltkontakte. Eine mehrfache Kodierung (oben, hinten, unten oder/und an der/den Seite/n) ist möglich. Die Abtasteinheit 32 kann beispielweise in eine Infusionspumpe 12 integriert sein.

Anstelle von einer mechanischen Abtastung der Schlüsselbart-Kontur 28 kann eine echte Schlüsselfunktion verwirklicht sein. Das heißt, dass die Schlauchklemme 2 nur bei passender Kodierung in eine zugehörige Aufnahme ("Schließzylinder") einschiebbar ist.

Obwohl die vorherige Beschreibung sich auf Schlauchklemmen 2 fokussiert hat, ist die Erfindung nicht darauf beschränkt. Die beschriebenen Kodierungen können vielmehr auch bei anderen Bestandteilen eines Infusionssets verwendet werden, um eine Identifikation zu ermöglichen und eine unerwünschte Nutzung zu vermeiden, etwa Infusionsschlauch, Tropfkammer, Luftfilter, Adapter, Nadel oder Katheter.

FIG. 5 gibt rein schematisch einen Überblick über eine beispielhafte Ausgestaltung des Gesamtsystems (Infusionspumpensystem 10), welches hier eine Infusionspumpe 12 mit integrierter Steuerung 34, zumindest einen mit einer Spektralkodierung, insbesondere einer Farbkodierung, und einem zusätzlichen Identifikationsmerkmal 14 ausgestatteten Artikel 4 eines Infusionssets sowie ein Sensorsystem 20 zur Erfassung der Spektralkodierung und des zusätzlichen Identifikationsmerkmals 14 aufweist. Das Sensorsystem 20 kann in die Infusionspumpe 12 integriert sein. Die Steuerung 34 kann zur Ansteuerung des Sensorsystems 20 und zur Auswertung der Messwerte ausgelegt sein. Basierend auf der sensorischen Auswertung steuert die Steuerung 34 den Betrieb der Infusionspumpe 12. Somit lässt sich der Artikel 4 erkennen und dediziert für eine entsprechende Anwendung vorsehen oder für eine ggf. parametrisierte Nutzung freischalten.

### Bezugszeichenliste

- 2: Schlauchklemme
- 4: Artikel
- 6: Grundkörper
- 8: Langloch
- 10: Infusionspumpensystem
- 12: Infusionspumpe
- 14: zusätzliches Identifikationsmerkmal
- 16: Stift oder Dorn
- 18: Aufdruck
- 20: Sensorsystem
- 22: Loch
- 24: Lochmuster
- 28: Schlüsselbart-Kontur
- 30: Stößel
- 32: Abtasteinheit
- 34: Steuerung

## Patentansprüche

1. Infusionspumpensystem (10), umfassend:
• einen Artikel (4) eines Infusionssets,
• eine Spektralkodierung, insbesondere eine Farbkodierung, auf dem Artikel (4) als Identifikationsmerkmal,
• mindestens ein zusätzliches Identifikationsmerkmal (14) auf dem Artikel (4), ausgewählt aus einer Gruppe bestehend aus geometrischen Merkmalen, Aufdrucken, Lochmustern oder spezifischen Konturformen,
• ein Sensorsystem (20), das sowohl die Spektralkodierung als auch das zusätzliche Identifikationsmerkmal (14) erkennen und auswerten kann.

2. Infusionspumpensystem (10) nach Anspruch 1, wobei der Artikel (4) eine Schlauchklemme (2), ein Infusionsschlauch, eine Tropfkammer, ein Luftfilter, ein Adapter, eine Nadel oder ein Katheter ist.

3. Infusionspumpensystem (10) nach Anspruch 3, wobei die Schlauchklemme (2) eine Schiebeklemme ist mit einem Grundkörper (6) und mit einem Langloch (8), durch welches ein Infusionsschlauch geführt wird.

4. Infusionspumpensystem (10) nach einem der vorangehenden Ansprüche, wobei das zusätzliche Identifikationsmerkmal (14) ein Stift oder Dorn (16) mit spezifischer Querschnittsform ist.

5. Infusionspumpensystem (10) nach einem der vorangehenden Ansprüche, wobei das zusätzliche Identifikationsmerkmal (14) ein Aufdruck (18), eine Prägung und/oder eine Gravur ist, der/die kodierte Informationen, bevorzugt in Form eines Strichcodes, QR-Codes oder anderen zweidimensionalen Codes, enthält.

6. Infusionspumpensystem (10) nach einem der vorangehenden Ansprüche, wobei das Sensorsystem (20) eine Kamera umfasst, die die Spektralkodierung und/oder das zusätzliche Identifikationsmerkmal (14) optisch erfasst (18).

7. Infusionspumpensystem (10) nach einem der vorangehenden Ansprüche, wobei das zusätzliche Identifikationsmerkmal (14) ein spezifisches Lochmuster (24) umfasst, das durch Löcher oder Durchgangsbohrungen oder Ausfräsungen im Artikel (4) gebildet wird.

8. Infusionspumpensystem (10) nach Anspruch 7, wobei das Sensorsystem (20) eine Lichtschranke oder ein photosensitives Array zur Erkennung des Lochmusters (24) umfasst.

9. Infusionspumpensystem (10) nach einem der vorangehenden Ansprüche, wobei das zusätzliche Identifikationsmerkmal (14) eine Schlüsselbart-Kontur (28) an der Außenkontur des Artikels (4) ist.

10. Infusionspumpensystem (10) nach Anspruch 9, wobei das Sensorsystem (20) eine mechanische Abtasteinheit (32) für die Schlüsselbart-Kontur (28) umfasst.

11. Infusionspumpensystem (10) nach einem der vorhergehenden Ansprüche, welches so ausgelegt ist, dass nur kompatible Artikel (4) mit spezifischer Spektralkodierung und/oder mit einem spezifischen zusätzlichem Identifikationsmerkmal (14) verwendet werden können.

12. Infusionspumpensystem (10) nach Anspruch 11, welches bei Erkennung eines inkompatiblen oder nicht kodierten Artikels (4) den Betrieb verweigert oder einschränkt.

13. Infusionspumpensystem (10) nach einem der vorhergehenden Ansprüche, wobei das zusätzliches Identifikationsmerkmal (14) auf dem Artikel (4) zur Individualisierung auf spezifische Anwendungen, Anwender oder Patienten dient.

14. Infusionspumpensystem (10) nach einem der vorhergehenden Ansprüche, wobei das Sensorsystem (20) in eine Infusionspumpe (12) integriert ist.
